(19) Europäisches Patentamt
European Patent Office
Office européen des brevets

(11) EP 3 654 944 B1

(12) EUROPEAN PATENT SPECIFICATION

(45) Date of publication and mention of the grant of the patent:
**26.11.2025 Bulletin 2025/48**

(21) Application number: **18835940.0**

(22) Date of filing: **18.07.2018**

(51) International Patent Classification (IPC):
***A61K 9/06*** *(2006.01)* ***B01J 23/42*** *(2006.01)*
***C01B 33/14*** *(2006.01)* ***A61L 26/00*** *(2006.01)*
***A61K 33/243*** *(2019.01)*

(52) Cooperative Patent Classification (CPC):
**A61L 26/0004; A61K 31/282; A61L 26/0066;**
A61L 2300/412; A61L 2400/12

(86) International application number:
**PCT/IB2018/055336**

(87) International publication number:
**WO 2019/016724 (24.01.2019 Gazette 2019/04)**

(54) **FORMULATION COMPRISING NANOSTRUCTURED, BIOCOMPATIBLE AND BIOCATALYTIC MATERIAL FOR THE TREATMENT OF WOUNDS AND INFECTIONS**

FORMULIERUNG MIT NANOSTRUKTURIERTEM, BIOKOMPATIBLEM UND BIOKATALYTISCHEM MATERIAL ZUR BEHANDLUNG VON WUNDEN UND INFEKTIONEN

FORMULATION COMPRENANT UNE MATIÈRE NANOSTRUCTURÉE, BIOCOMPATIBLE ET BIOCATALYTIQUE POUR LE TRAITEMENT DE PLAIES ET D’INFECTIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.07.2017 US 201762534747 P**

(43) Date of publication of application:
**27.05.2020 Bulletin 2020/22**

(73) Proprietor: **Nanotess Inc.**
**Calgary AB T2E6A8 (CA)**

(72) Inventor: **LÓPEZ-GOERNE, Tessy María**
**04120 Mexico City (MX)**

(74) Representative: **Schneiter, Sorin et al**
**ABREMA SA**
**Avenue du Théâtre 16**
**1005 Lausanne (CH)**

(56) References cited:
**WO-A1-2011/045627**
**WO-A1-2011/045627**
**KR-B1- 101 020 041**
**US-A1- 2007 122 463**
**US-A1- 2008 089 839**
**US-B2- 8 026 382**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 654 944 B1

## Description

### FIELD OF THE INVENTION

This invention relates to a formulation comprising a nanostructured biocompatible and biocatalytic material to be employed in the treatment and care of wounds and infections of the skin of animals and humans.

### BACKGROUND

Wounds and infections are a serious health problem, specially for patients sufferinf Diabetes Mellitus. Diabetic patients may suffer with a diminished neurocapacity and immune response and thereby are prone to develop wounds and those wounds may easily get infected, these wounds and infections are usually found in the foot of diabetic patients which results in whats is called "Diabetic Foot". Although some treatments exist for this condition such as the use of normal antibiotics, surgery, and other more complex treatments as use of growth factors (bFGF). For less invasive procedures, bandages made of biomaterial with a susteained release capability containing bio-active substances are also used However, the current treatments are either complex, expensive, invasive and/or their results are not ideal.

The present invention refers to a formulation comprising nanostructured, biocompatible and biocatalytic material comprising a solid acid consisting of mixed oxides of silica and titania ($TiO_2$-$SiO_2$); supporting in its dispersed matrix: copper, silver, gold, iron, rutenium, palladium, zinc, manganese, iridium and/or platinum metals at minimal concentrations, for use in the treatment of wounds and infections.

In the past decade, scientists' ability to manipulate materials at the molecular and atomic levels using nanotechnology has moved from science fiction into scientific fact. Now, nanotechnology is being developed to prevent, diagnose, and treat infectious diseases, with some products in or nearing clinical trial stage. Progress in this field is exponential. Cross-disciplinary nanoscience and nanomedicine researches involving chemists, physicists, biologists and engineers is concerned about the need for developing environmentally friendly and sustainable methods for the synthesis of nanomaterials. There is a current drive to integrate all the green chemistry approaches to design environmentally benign materials and processes. Rapid developments are taking place in the synthesis of biocompatible mixed oxides or simple oxides metallic and bimetallic nanomaterials and their surface modification for bioactivity and nanomedical applications. Biosynthesis of nanoparticles as an emerging highlight of the intersection of nanotechnology and biotechnology has received increased attention due to a growing need to develop environmentally benign technologies in material syntheses. Biomolecules as reluctant are found to have a significant advantage over their counterparts as protecting agents.

The properties of the materials can change notably when its particle size is reduced to particles in the scale of nanometers. In materials science "particle" is a general term to describe small solid objects with sizes ranging from atomic scale ($10^{-10}$ m) to the microscopic scale ($10^{-3}$ m). However, the particle size often lies between $10^{-9}$-$10^{-5}$ m. Large particles (> $10^{-6}$) are commonly called grains (i. e. zeolites, carbon, Raney metals) and small particles (<15 nm) are frequently added (metals) to mixed oxides, i.e. $TiO_2$-$SiO_2$, $SiO_2$ or $TiO_2$. All materials consist of grains (particles) formed by the agglomeration of nanoparticles.

In conventional materials the grains have a size ranging from 100 micrometers to millimeters (mm), while the Nanomaterials particles are in the order of a one billionth of a meter ($10^{-9}$). A nanometer is around the average diameter of human hair. The radius of an atom is 1 to 3 Angstrom (Å) and a nanometer is equal to 10 Å. Nanomaterials are rigid solid, resistant and are ductile at high temperatures, are resistant to degradation, erosion and corrosion, are also very chemically active. The physical and chemical properties of each nanomaterial or nanostructured material are determined by the type of interactions compounds that functionalized the nanoparticles; as well the electronic density and hydroxyl concentration in the net have an important role in the DNA cracking.

One of the areas in which nanoparticles have increased the importance is in the field of catalysis and biocatálisis, in order to obtain a distribution of particles with well defined shape and size to improve the catalytic activity. In particular, it is necessary to obtain highly dispersed particles in which most of the atoms are located on the surface. The structure includes a solid area, pore size, as well as the shape and volume of the pores. These parameters are important in catalysis and in biocatalysis, because they are responsible to increase the reaction rate. Although the catalytic activity can be related directly with its total contact area between the material and the reactive to catalyze, the determination of the area is generally considered to be an important requirement in the characterization of the biocatalysts. Furthermore, it is necessary to specify the nature of the structure of the pores as they control the transport of reagents and products in the catalytic reaction. In many cases, the course of the reaction is influenced by the size and shape of the pore but the particle size of the support and the metals are more definitively.

Titanium dioxide occurs in nature in three crystalline phases; anatase, rutile and brookite. The anatase and brookite can transform into rutile at high temperatures. The anatase to rutile can be irreversibly transformed by heating. There are several factors that influence the phase shift, such as particle size, crystal morphology, but in particular influence of the poisoning ions to the network. The literature indicates that the three phases, the anatase has a great chemical

stability, resistance to corrosion, is inert from biological agents and has high specific surface area. However, the commercial titania Is a mix (Degussa P25) and contains 60 to 80% anatase. The problem only to produce the anatase phase is due to the rutile phase is thermodynamically more stable. Therefore by the sol-gel process is possible to obtain pure anatase and pure rutile. The structure of anatase and rutile are tetragonal, whereas brookite is orthorhombic, each titanium atom is attached to 6 oxygen atoms almost equidistant and each oxygen atom is linked to three atoms of titanium Some antecedents of similar materials can be found in: WO 2006/080241, WO 2004/089839 as well as:

U.S. Pat. No. 6 124 367. This patent protects reservoirs used in the Fischer Tropsch reactions from sintering by imparting a higher degree of mechanical strength to the reservoir. It incorporates $SiO_2$ and $Al_2O_3$ into the reservoir and claims a rutile-anatase ratio of 1/9. It is a porous reservoir with either a spherical or a cylindrical shape. It is made by extrusion, spray drying or tableting.

U.S. Pat. No. 6117814**.** This patent describes a titania reservoir which also incorporates silica and alumina as a binder into the structure. The purpose of the binder is to impart better mechanical properties to the reservoir. The size range of this reservoir is from between 20 to 120 microns. The reservoir is approximately 50% binder, which is fabricated by a sol-gel process.

U.S. Pat No 6087405**.** This patent describes a reservoir to be used in a Fischer Tropsch gas synthesis reaction. The reservoir incorporates group VII metals into its structure. The rutile-anatase ratio in the structure is a distinguishing feature of this patent.

**Centre National De La Recherche Scientifique.** WO/2003/064324 The invention relates to a titanium oxide-based polymer composition. The inventive composition comprises a $TiO_x(OH)_y(H_2O)_z$ (x+y+z=3) titanium oxide-based polymer in the form of a gel or sol. Said polymer, which has a one-dimensional (1D) structure, is made from concentrically-wound fibres having a periodicity, which is deduced from the spacing between said fibres, of between 3.5 Å and 4 Å. Each fibre comprises $TiO_6$ octahedrons and each $TiO_6$ octahedron shares two opposite edges with two adjacent octahedrons (2 x 2.92 Å) in order to form infinite chains which develop along the axis of a fibre. According to the invention, two adjacent chains form double lines as a result of the shared edges (2 x 3.27 Å). The inventive polymer is suitable for use as a photosensitive element in a photovoltaic cell, such as a sunscreen for a window.

WO/2006/079757**.** Method of preparing stable oxide ceramic precursor sol-gel solutions based on lead, titanium, zirconium and lanthanide(s) and method of preparing said ceramic. The invention relates to a method of preparing a stable oxide ceramic precursor sol-gel solution based on lead, titanium, zirconium and lanthanide(s). The invention comprises the following successive steps consisting in: a) preparing a sol-gel solution by bringing a molecular lead precursor, a molecular titanium precursor, a molecular zirconium precursor and a molecular lanthanide precursor into contact with a medium comprising a diol solvent and optionally an aliphatic mono-alcohol; b) leaving the solution thus obtained to stand for a sufficient period of time in order to obtain a solution having an essentially-constant viscosity; and c) diluting the solution obtained in the preceding step with a diol solvent identical to that used in step a or a solvent that is miscible with said solvent, at a predetermined rate. The invention can be used to prepare an oxide ceramic material comprising lead, a lanthanide metal, titanium and zirconium.

WO2007/141590**.** Sol-gel nanostructured titania reservoirs for use in the controlled release of drugs in the central nervous system and method of synthesis. The invention is related to a sol-gel nanostructured titania reservoir and its synthesis which is biocompatible with brain tissue. The pore size distribution, crystallite size and the extent of the crystalline phase distribution of anatase, brookite and rutile can be fully controlled. This device may be used to contain neurological drugs. It may be inserted directly into brain tissue for the purpose of the controlled time release of drugs over a period of from 6 months to three years.

WO93/21969**.** Novel coating materials for biomedical applications, particularly for use on biomedical implants, the coating material containing gel-derived titania where the material is capable of inducing calcium phosphate formation onto its surface under in vitro conditions, e.g. in a simulated body fluid and/or under in vivo conditions, processes for the preparation of the coating materials as well as their use in biomedical implant technology.

WO 2011/045627**:** Viricide agents having nanostructured biocatalysts materials of titanium dioxide ($TiO_2$) and silicium dioxide $SiO_2$) matrix with platinum and iridium modified and dispersed on the surface of the matrix, and a method of making same.

These nanostructured products have thereby an innovative use and, in particular, provide the foundations of Catalytic Nanomedicine, to completely eliminate amputation and improve quality of life.

## SUMMARY OF THE INVENTION

The invention provides a nanoparticle formulation comprising a nanostructured, biocompatible and biocatalytic inorganic network gel solid acid consisting of (i) mixed oxides of silica and titania ($TiO_2$-$SiO_2$) and supporting in its dispersed matrix, (ii) one or more functional groups selected from the group consisting of a sulfate, an amine and a phosphate, and (iii) coordinated metallic atoms selected from the group consisting of copper, silver, gold, iron, rutenium, zinc, palladium, manganese, iridium and platinum metals at minimal concentrations, for use in the treatment of wounds

and infections of skin in animals and humans.

## DESCRIPTION OF THE DRAWINGS

**Figure 1** is the infrared spectrum with Fourier transform in the interval of 4000-400 $cm^{-1}$ of the nanostructured biocompatible and biocatalytic material prepared by the sol-gel method.

**Figure 2** is a photograph at three different resolutions, obtained by a scanning electron microscope of the nanostructured biocompatible and biocatalytic material prepared by the sol-gel method.

**Figure 3** are photographs at three different times after application of : 0 days, 8 days and 20 days, showing effects of the formulation comprising the nanostructured biocompatible and biocatalytic material.

## DETAILED DESCRIPTION

The presention invention refers to a formulation comprising a new nanostructured, biocatalytic, biocompatible and non-toxic material obtained by the sol-gel chemical synthesis method. The sol-gel methodology is used to control the physico-chemical properties of the material in a thin, nanometric size and with a wide surface area. Biocompatibility is achieved by the superficial incorporation of functional groups of sulfates, amines and phosphates that allow the easy interaction of the cellular membrane nanoparticle, and thus easily enter the cell and perform the necessary (catalytic) specific activity. The material is a nanoparticle characterízed by being a solid acid consisting of mixed oxides of silica and titania ($TiO_2$-$SiO_2$), further specification of the nanoparticle can be observed in figures 1 and 2 that show the infrared spectrum with Fourier transform in the interval of 4000-400 $cm^{-1}$ of the biocatalyzer prepared by the sol-gel method; supporting in its dispersed matrix: copper, silver, gold, iron, rutenium, palladium, zinc, manganese, iridium and/or platinum metals at minimal concentrations. The components that integrate the nanoparticles are very stable and have been tested experimentally extensively in vitro and in vivo, and there is scientific literature evidence of its biocompatibility and low cytotoxicity.

### Sol-gel process using metal alkoxides.

At the functional group level, three reactions are generally used to describe the sol-gel process: hydrolysis, alcohol condensation, and water condensation. However, the characteristics and properties of a particular sol-gel inorganic network are related to a number of factors that affect the rate of hydrolysis and condensation reactions, such as, pH, temperature and time of reaction, reagent concentrations, catalyst nature and concentration, $H_2O$/M molar ratio (R), aging temperature and time, and drying. Of the factors listed above, pH, nature and concentration of catalyst, $H_2O$/M molar ratio (R), and temperature have been identified as most important. Thus, by controlling these factors, it is possible to vary the structure and properties of the sol-gel-derived inorganic network over wide ranges. For example, Sakka et al. observed that the hydrolysis of TEOS utilizing R values of 1-2 and 0.01 M HCl as a catalyst yields a viscous, spinnable solution. It was further shown, that these solutions exhibited a strong concentration dependence on the intrinsic viscosity and a power law dependence of the reduced viscosity on the number average molecular weight[31-34]:

$$[n] = k(Mn)a \qquad (1)$$

Values for a ranged from 0.5 to 1.0, which indicates a linear or lightly branched molecule or chain.

Values of "a" in eq. 1 ranged from 0.1 to 0.5, indicating spherical or disk shaped particles. These results are consistent with the structures which emerge under the conditions employed by the Ströber process, for preparing $SiO_2$ powders. It was further shown that with hydrolysis under basic conditions and R values ranging from seven (7) to twenty-five (25), monodisperse, spherical particles could be produced.

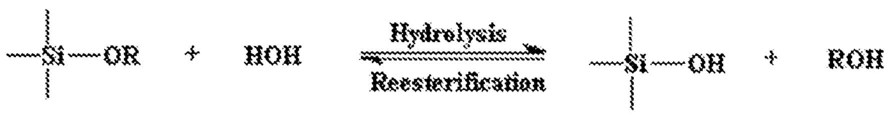

I

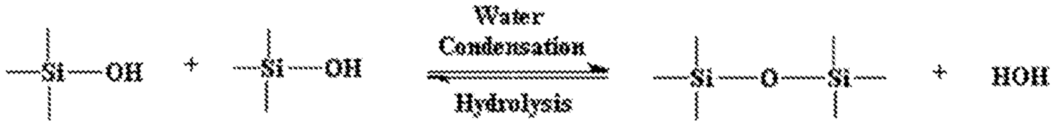

2a

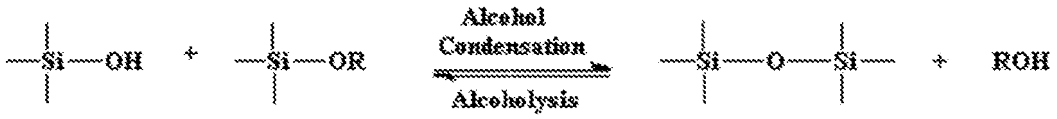

2b

Generally speaking, the hydrolysis reaction (Eq. 2), through the addition of water, replaces alkoxide groups (OR) with hydroxyl groups (OH). Subsequent condensation reactions are made, involving the silanol groups (Si-OH) produce siloxane bonds (Si-O-Si) plus the by-products water or alcohol in the case of silica. Under most conditions, condensation commences before hydrolysis is complete. However, conditions such as, pH, $H_2O$/Si molar ratio (R), and catalyst can force completion of hydrolysis before condensation begins. Additionally, because water and alkoxides are immiscible, a mutual solvent is utilized. With the presence of this homogenizing agent, alcohol, hydrolysis is facilitated due to the miscibility of the alkoxide and water. As the number of siloxane bonds increases, the individual molecules are bridged and jointly aggregate in the sol. When the sol particles are aggregate, or inter-knit into a network, a gel is formed. Upon drying, trapped volatiles (water, alcohol, etc.) are driven off and the network shrinks as further condensation can occur. It should be emphasized, however, that the addition of solvents and certain reaction conditions may promote esterification and depolymerization reactions. The hydrolysis/condensation reaction follows two different mechanisms, which depend of the coordination of metallic central atom. When the coordination number is satisfied the hydrolysis reaction occurs by nucleophilic substitution ($S_n$):

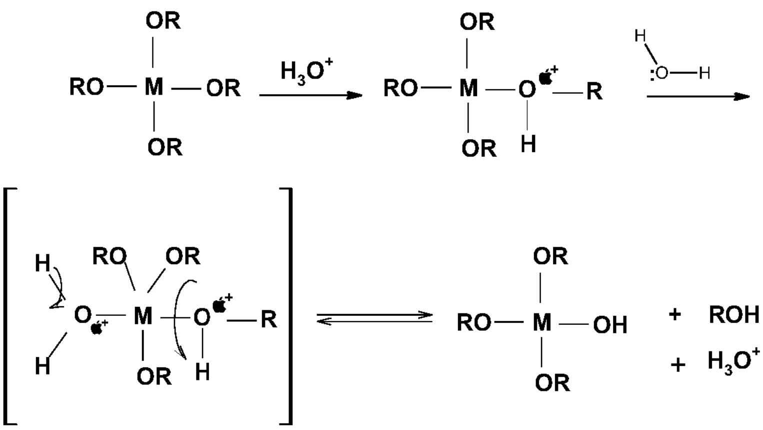


**Hydrolysis reaction via nucleophilic substitution ($S_n$).**

When the coordination number is major, the hydrolysis reaction takes place by nucleophilic addition:

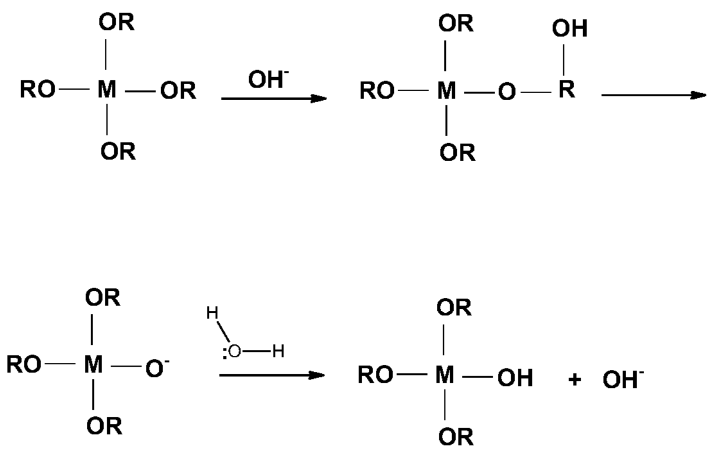

## Hydrolysis reaction via nucleophilic addition ($A_n$).

These mechanisms need that the oxygen coordination is increased from 2 to 3, the additional bond generation involves one electron pair of the oxygen and the new bond can be equivalent to the other bonds. During the condensation step an enormous concentration of hydroxyl groups are formed. This OH can be linked between the metallic atoms or only be simple -OH ligand in the surface.

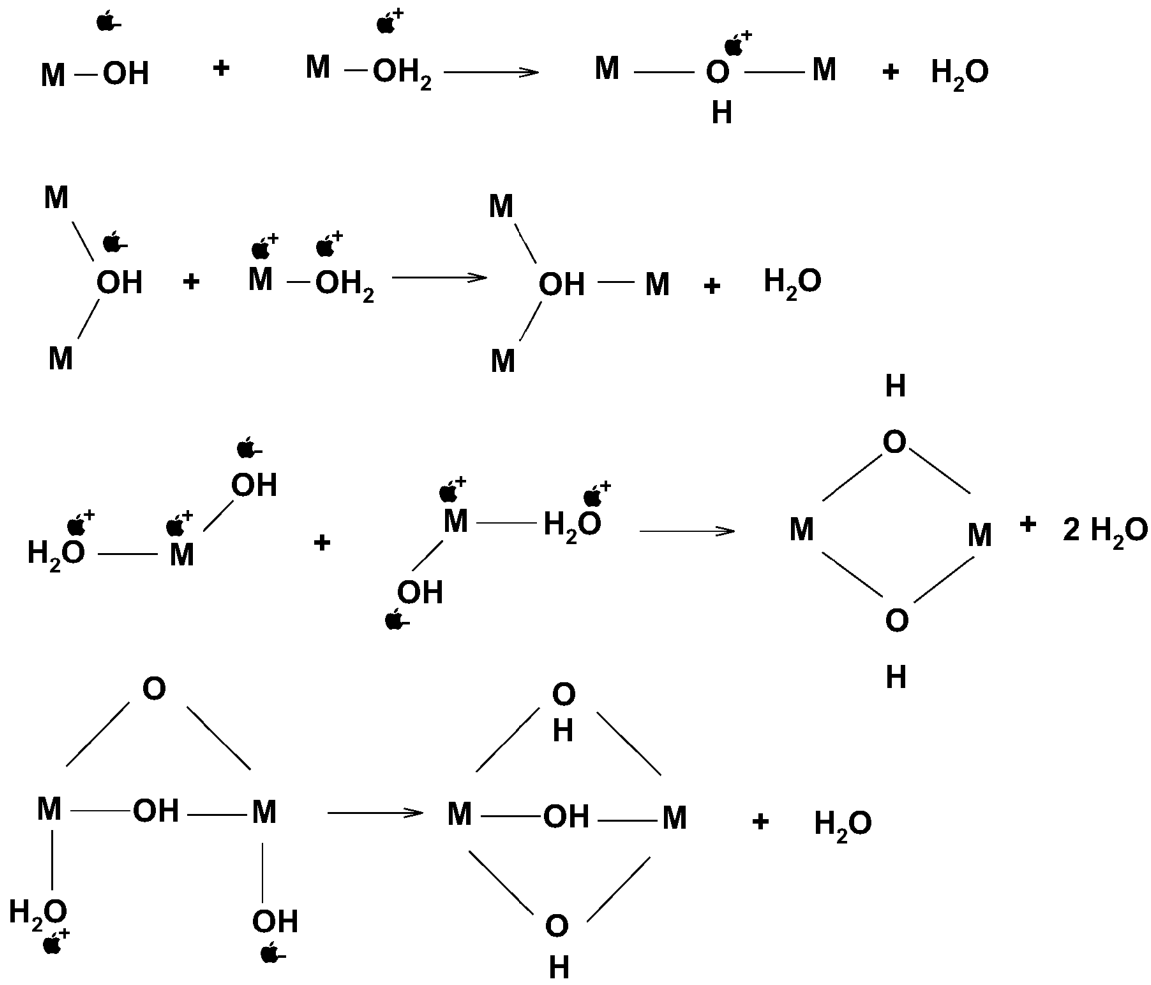

## Condensation step of the sol-gel method

Acid-Catalyzed Mechanism

Under acidic conditions, it is likely that an alkoxide group is protonated in a rapid first step. Electron density is withdrawn from the silicon atom, making it more electrophilic and thus more susceptible to attack from water. This results in the formation of a penta-coordinate transition state with significant $SN_2$-type character. 13 The transition state decays by displacement of an alcohol and inversion of the silicon tetrahedron, using silica as example:

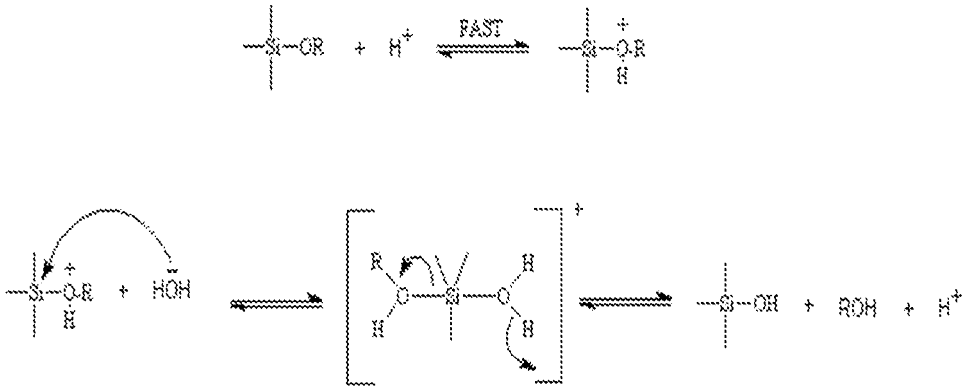


## Acid-Catalyzed Hydrolysis

Base-Catalyzed Mechanism

Base-catalyzed hydrolysis of silicon alkoxides proceeds much more slowly than acid-catalyzed hydrolysis at an equivalent catalyst concentration. Basic alkoxide oxygens tend to repel the nucleophile, -OH. However, once an initial hydrolysis has occurred, following reactions proceed stepwise, with each subsequent alkoxide group more easily removed from the monomer then the previous one. Therefore, more highly hydrolyzed silicones are more prone to attack. Additionally, hydrolysis of the forming polymer is more sterically hindered than the hydrolysis of a monomer. Although hydrolysis in alkaline environments is slow, it still tends to be complete and irreversible. Thus, under basic conditions, it is likely that water dissociates to produce hydroxyl anions in a rapid first step. The hydroxyl anion then attacks the silicon atom. Again, an $SN_2$-type mechanism has been proposed in which the -OH displaces -OR with inversion of the silicon tetrahedron.

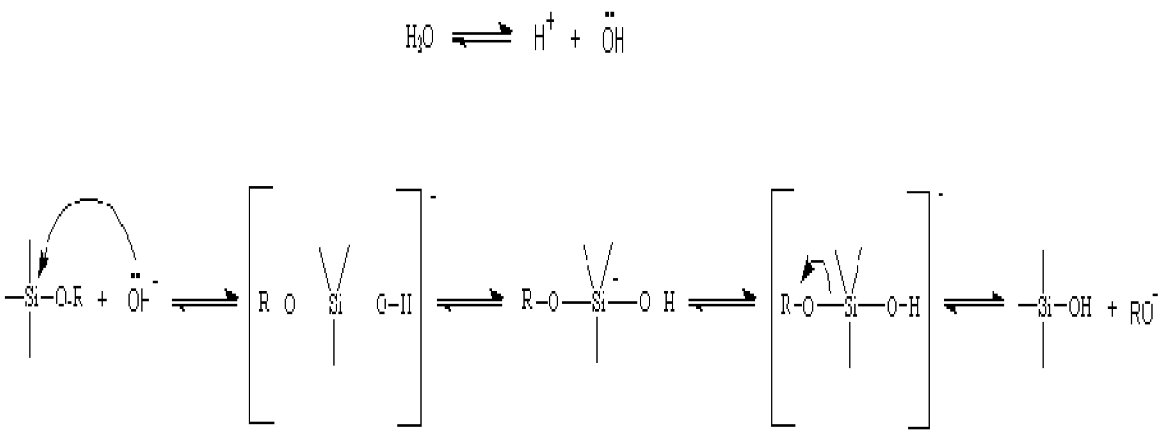

## Base-Catalyzed Hydrolysis

The nanoparticle formulation was invented to counteract one of the most terrible and disabling consequences of Diabetes Mellitus, such as diabetic foot. Diabetic foot consists of a condition of the blood vessels and nerves, leading to inadequate vascular and arterial irrigation, as well as lack of sensation, which promotes the formation of ischemic and / or neuropathic ulcers on the soles of the feet. Added to this, in many cases, the ulcers are infected by a variety of microorganisms which further hinder the closure of the lesion and can lead to the affection of bone and tendons. Both in the animal model and in a clinical trial with patients, antibacterial and healing effects have been demonstrated, promoting the granulation, regeneration, angiogenesis and epithelization of the tissues as shown in figure 3.

This nanoparticle is also capable of promoting the closure of other types of wounds, for example, fistulas, surgical wounds and burns; among others.

The nanostructured material is easy to apply by medical personnel with basic training in wound care. The procedure consists of: 1) washing the affected area with sterile or boiled water and surgical soap; 2) carving with a gauze and, if necessary, debride; 3) drying; 4) applying the nanoparticles directly on the lesion in a gel, powder or powder dissolved in saline solution formulation; and 5) dressing the wound. The treatment is applied every third day, except when the patient treated is very serious and / or under the consideration of the treating physician. It can be done daily.
It is important to note that no serious short-term side effects were observed (n = 40 patients).

## EXAMPLES

Example 1

1.6871 g Of acetil platinum acetonate ($Pt(acac)_2$) were mixed with 246 ml of acetone. The mixture was maintained under agitation until the platinum complex was disolved. To this mixture, 1.2501 g of GABA previously dissolved in 20 ml of deionized water was added. pH was adjusted to 3 with phosphoric acid. Simultanoeusly, 89 ml of TEOS and 9.8 ml of titanium butoxide dissolved in 70 ml of deionized water were added. Everything was maintained under agitation and at room temperature until de gel was formed.

Example 2

1.6871 g Of acetil platinum acetonate ($Pt(acac)_2$) were mixed with 246 ml of acetone. The mixture was maintained under agitation until the platinum complex was disolved. To this mixture, 1.2501 g of glutamic acid previously dissolved in 20 ml of deionized water was added. pH was adjusted to 3 with phosphoric acid. Simultanoeusly, 89 ml of TEOS and 9.8 ml of titanium butoxide dissolved in 15 ml of absolute ethanol. 1.7203 g of ammonium sulphate dissolved in 70 ml of deionized water was added. Everything was maintained under agitation and at room temperature until de gel was formed.

Example 3

1.6871 g Of acetil platinum acetonate ($Pt(acac)_2$) were mixed with 246 ml of acetone. The mixture was maintained under agitation until the platinum complex was disolved. To this mixture, 1.2501 g of GABA previously dissolved in 40 ml of deionized water was added. pH was adjusted to 3 with phosphoric acid. Simultanoeusly, 89 ml of TEOS and 9.8 ml of titanium butoxide dissolved in 15 ml of absolute ethanol were added. 1.7203 g of ammonium sulphate dissolved in 140 ml of deionized water was added. Everything was maintained under agitation and at room temperature until de gel was formed.

## Claims

1. A nanoparticle formulation comprising a nanostructured, biocompatible and biocatalytic inorganic network gel solid acid consisting of (i) mixed oxides of silica and titania (TiO2-SiO2) and supporting in its dispersed matrix, (ii) one or more functional groups selected from the group consisting of a sulfate, an amine and a phosphate, and (iii) coordinated metallic atoms selected from the group consisting of copper, silver, gold, iron, ruthenium, zinc, palladium, manganese, iridium and platinum metals at minimal concentrations for use in the treatment of wounds and infections of skin in animals and humans.

2. The nanoparticle formulation of claim 1, wherein the coordinated metal atoms in the inorganic network gel are platinum.

3. The nanoparticle formulation of claim 1, wherein the coordinated metal atoms in the inorganic network gel are copper.

4. The nanoparticle formulation of any one of claims 1 to 3, wherein the wounds and infections are consequences with Diabetes Mellitus.

5. The nanoparticle formulation of any one of claims 1 to 4, wherein the wounds and infections are selected from diabetic foot ulcer, fistula, surgical wound or burns.

6. The nanoparticle formulation of any one of claims 1 to 5, wherein the inorganic network gel is for application directly on a lesion in a gel, powder or powder dissolved in saline solution.

7. The nanoparticle formulation of any one of claims 1 to 3, wherein the inorganic network gel is for promoting one or more of granulation, regeneration, angiogenesis, and epithelization of the tissues in Diabetic Mellitus ulcers.

## Patentansprüche

1. Nanopartikelformulierung, die ein nanostrukturiertes, biokompatibles und biokatalytisches anorganisches Feststoffsäure-Netzwerkgel umfasst, das gebildet ist aus (i) gemischten Oxiden von Siliziumdioxid und Titandioxid (TiO2-SiO2), und in ihrer dispergierten Matrix (ii) einer oder mehreren funktionellen Gruppen, ausgewählt aus der Gruppe gebildet aus einem Sulfat, einem Amin und einem Phosphat, und (iii) koordinierten Metallatomen, ausgewählt aus der Gruppe gebildet aus Kupfer, Silber, Gold, Eisen, Ruthenium, Zink, Palladium, Mangan, Iridium und Platinmetallen in minimalen Konzentrationen zur Verwendung bei der Behandlung von Wunden und Infektionen der Haut bei Tieren und Menschen.

2. Nanopartikelformulierung nach Anspruch 1, wobei die koordinierten Metallatome in dem anorganischen Netzwerkgel Platin sind.

3. Nanopartikelformulierung nach Anspruch 1, wobei die koordinierten Metallatome in dem anorganischen Netzwerkgel Kupfer sind.

4. Die Nanopartikelformulierung nach irgendeinem der Ansprüche 1 bis 3, wobei die Wunden und die Infektionen Folgen von Diabetes Mellitus sind.

5. Die Nanopartikelformulierung nach irgendeinem der Ansprüche 1 bis 4, wobei die Wunden und die Infektionen aus diabetischem Fußgeschwür, Fistel, chirurgischer Wunde oder Verbrennungen ausgewählt sind.

6. Nanopartikelformulierung nach irgendeinem der Ansprüche 1 bis 5, wobei das anorganische Netzwerkgel zur direkten Anwendung eines Gels, Pulvers oder in Kochsalzlösung gelösten Pulvers auf eine Läsion bestimmt ist.

7. Die Nanopartikelformulierung nach irgendeinem der Ansprüche 1 bis 3, wobei das anorganische Netzwerkgel zur Förderung der Granulation, Regeneration, Angiogenese und/oder Epithelisierung des Gewebes in diabetischen Mellitusgeschwüren dient.

## Revendications

1. Formulation de nanoparticules comprenant un gel de réseau inorganique d'un acide solide, le gel étant nanostructuré, biocompatible et biocatalytique et constitué (i) d'oxydes mixtes de silice et de titane (TiO2-SiO2) et supportant dans sa matrice dispersée, (ii) un ou plusieurs groupes fonctionnels choisis dans le groupe constitué par un sulfate, une amine et un phosphate, et (iii) des atomes métalliques coordonnés choisis dans le groupe constitué par les métaux cuivre, argent, or, fer, ruthénium, zinc, palladium, manganèse, iridium et platine à des concentrations minimales pour une utilisation dans le traitement des plaies et des infections de la peau chez l'animal et l'homme.

2. Formulation de nanoparticules selon la revendication 1, dans laquelle les atomes métalliques coordonnés dans le gel de réseau inorganique sont le platine.

3. Formulation de nanoparticules selon la revendication 1, dans laquelle les atomes métalliques coordonnés dans le gel de réseau inorganique sont le cuivre.

4. Formulation de nanoparticules selon l'une quelconque des revendications 1 à 3, dans laquelle les plaies et les infections sont des conséquences du diabète sucré.

5. Formulation de nanoparticules selon l'une quelconque des revendications 1 à 4, dans laquelle les plaies et les infections sont choisies parmi l'ulcère du pied diabétique, les fistules, les plaies chirurgicales ou les brûlures.

6. Formulation de nanoparticules selon l'une quelconque des revendications 1 à 5, dans laquelle le gel de réseau inorganique est destiné à être appliqué directement sur une lésion sous forme de gel, de poudre ou de poudre dissoute dans une solution saline.

**7.** Formulation de nanoparticules selon l'une quelconque des revendications 1 à 3, dans laquelle le gel de réseau inorganique favorise une ou plusieurs de la granulation, la régénération, l'angiogenèse et l'épithélisation des tissus dans les ulcères du diabète sucré.

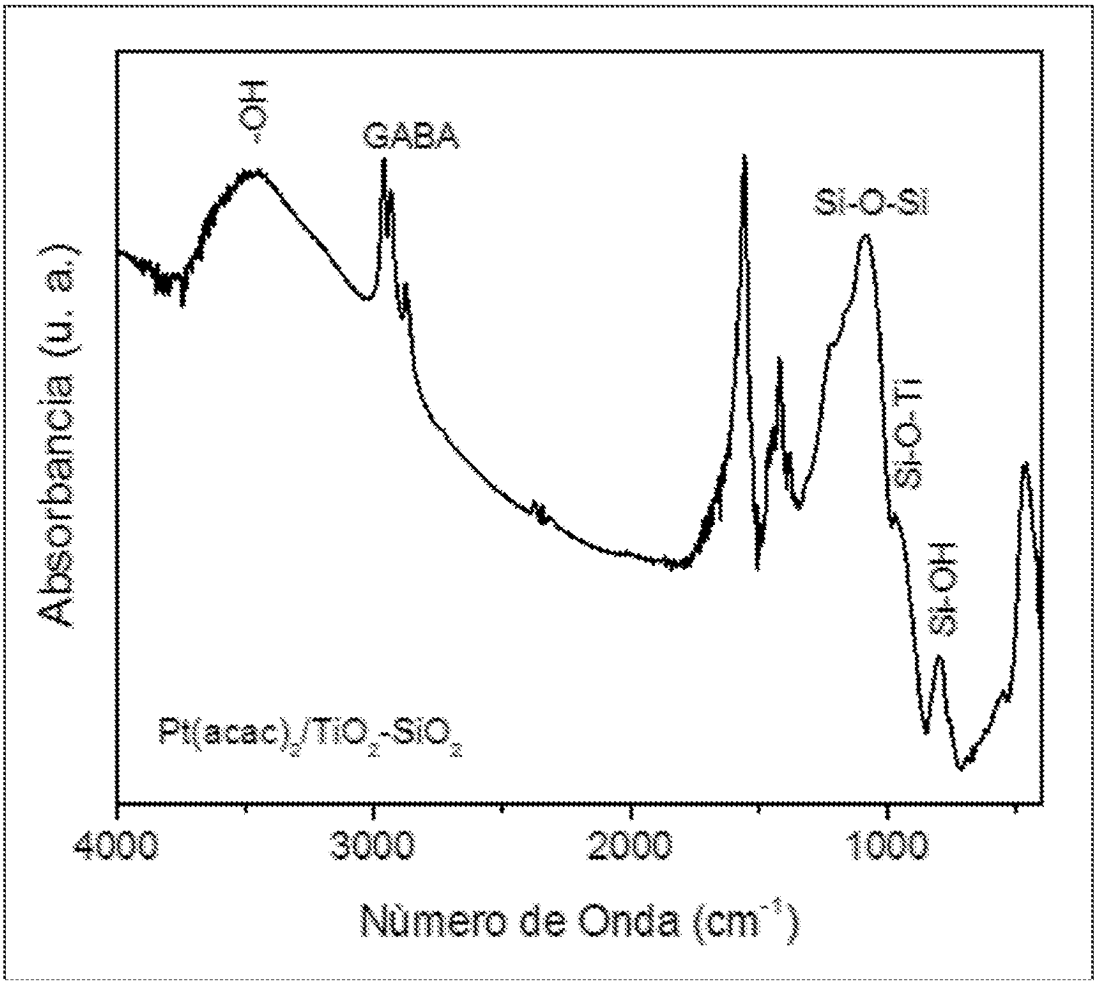
-OH
GABA
Si-O-Si
Si-O-Ti
Si-OH
Absorbancia (u. a.)
Pt(acac)2/TiO2-SiO2
4000
3000
2000
1000
Nùmero de Onda (cm-1)

**Figure 1**

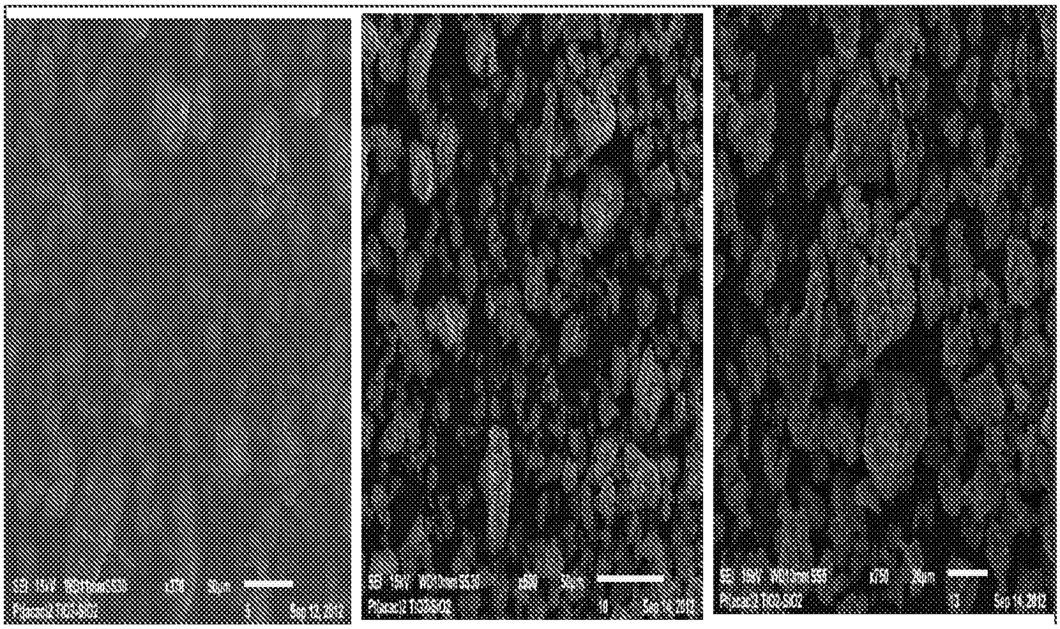

Figure 2

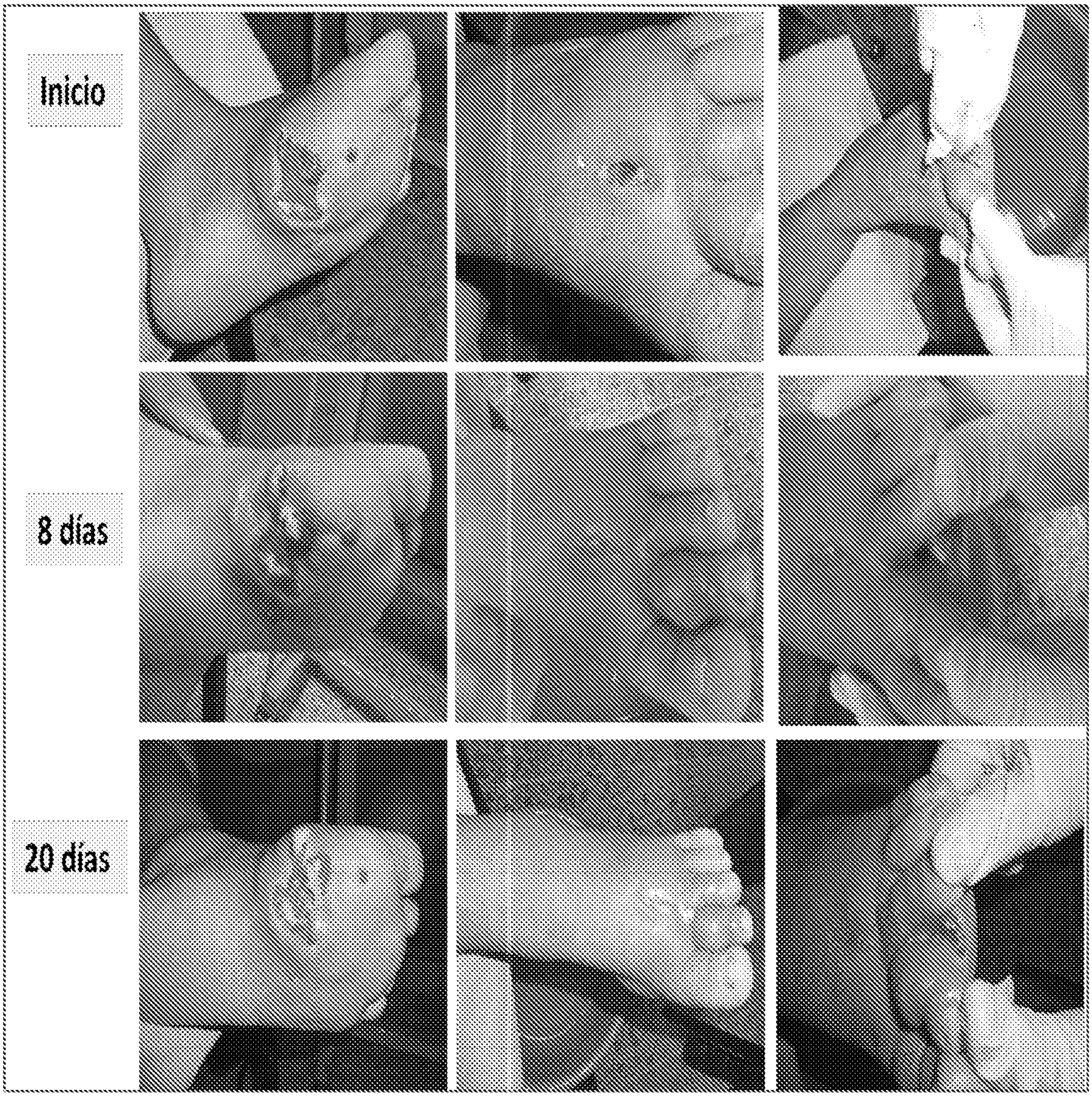


Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006080241 A
- WO 2004089839 A
- US 6124367 A
- US 6117814 A
- US 6087405 A
- WO 2003064324 A
- WO 2006079757 A
- WO 2007141590 A
- WO 9321969 A
- WO 2011045627 A